(19) Europäisches Patentamt
European Patent Office
Office européen des brevets

(11) Veröffentlichungsnummer : **0 071 096**
**B1**

(12) **EUROPÄISCHE PATENTSCHRIFT**

(45) Veröffentlichungstag der Patentschrift : **07.11.84**

(51) Int. Cl.³ : **C 07 C125/08**, C 07 C127/22, A 01 N 47/34, A 01 N 47/40

(21) Anmeldenummer : **82106338.5**

(22) Anmeldetag : **15.07.82**

(54) **N-Aminomethyl-halogenacetanilide, Verfahren zu ihrer Herstellung und ihre Verwendung zur Bekämpfung unerwünschten Pflanzenwuchses.**

(30) Priorität : **31.07.81 DE 3130302**

(43) Veröffentlichungstag der Anmeldung : **09.02.83 Patentblatt 83/06**

(45) Bekanntmachung des Hinweises auf die Patenterteilung : **07.11.84 Patentblatt 84/45**

(84) Benannte Vertragsstaaten : **AT BE CH DE FR GB IT LI NL SE**

(56) Entgegenhaltungen :
**EP-A- 0 012 428**
**EP-A- 0 033 114**
**US-A- 3 901 685**

(73) Patentinhaber : **BASF Aktiengesellschaft**
**Carl-Bosch-Strasse 38**
**D-6700 Ludwigshafen (DE)**

(72) Erfinder : **Eicken, Karl, Dr.**
**Waldstrasse 63**
**D-6706 Wachenheim (DE)**
Erfinder : **Rohr, Wolfgang, Dr.**
**In der Dreispitz 13**
**D-6706 Wachenheim (DE)**
Erfinder : **Wuerzer, Bruno, Dr., Dipl.-Landwirt**
**Ruedigerstrasse 13**
**D-6701 Otterstadt (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 071 096 B1

Jouve, 18, rue St-Denis, 75001 Paris, France

**Beschreibung**

Die Erfindung betrifft N-Aminomethyl-halogenacetanilide, Verfahren zu ihrer Herstellung und Herbizide, die diese Verbindungen enthalten.

Als Halogenacetanilide mit herbiziden Eigenschaften sind u. a. die Verbindungen 2,6-Diethyl-N-(methoxymethyl)-2'-chloracetanilid (US-A-3 442 945) und 2-Methyl-6-ethyl-N-(2-methoxy-1-methylethyl)-2'-chloracetanilid (DE-A-23 28 340) bekannt. Ferner ist bekannt, daß Chloracetanilide mit über Methylen verknüpften Amidgruppen eine herbizide Wirkung haben (DE-A-22 26 543, US-A-4 097 262, US-A-3 901 685).

Es wurde nun gefunden, daß sich substituierte N-Aminomethyl-halogenacetanilide der Formel

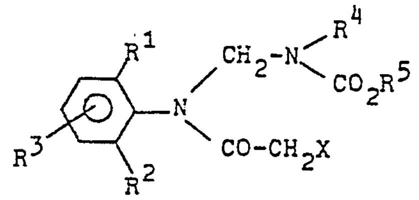

(I),

in der

$R^1$ $C_1$-$C_2$-Alkyl,
$R^2$ $C_1$-$C_2$-Alkyl,
$R^3$ Wasserstoff oder $C_1$-$C_4$-Alkyl,
$R^4$ den Rest $-CO-NH_2$,
$R^5$ $C_1$-$C_6$-Alkyl und
X Halogen

bedeuten, bei guter herbizider Wirksamkeit besonders durch ihre Verträglichkeit für Kulturpflanzen auszeichnen.

Unter Alkyl für die Reste $R^1$, $R^2$, $R^3$ oder $R^5$ sind je nach Zahl der genannten Kohlenstoffatome beispielsweise folgende Gruppen zu verstehen : Methyl, Ethyl, n-Propyl, iso-Propyl, n-, iso-, sec- oder tert.-Butyl, n-Pentyl, n-Hexyl und ihre Isomeren. X steht für Chlor, Brom oder Jod. Bevorzugte Bedeutung für X ist Chlor.

Man erhält die N-Aminomethyl-halogenacetanilide der Formel I durch Verseifung von N-Aminomethyl-halogenacetaniliden der Formel

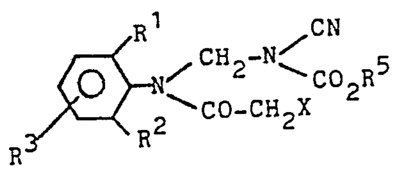

(IV),

in der $R^1$, $R^2$, $R^3$, $R^5$ und X die obengenannten Bedeutungen haben, mit Mineralsäure, in Gegenwart eines Alkohols und gegebenenfalls in Gegenwart eines inerten Verdünnungsmittels. Die Umsetzung kann in einem Verdünnungsmittel durchgeführt werden, gegenüber dem die Reaktionspartner inert sind. Als Verdünnungsmittel kommen z. B. aromatische Kohlenwasserstoffe, wie Toluol, Xylol, halogenierte aliphatische oder aromatische Kohlenwasserstoffe, wie Chlorbenzol, Chloroform, Methylenchlorid, Ether, wie Diethylether, Tetrahydrofuran, oder Gemische dieser Lösungsmittel in Betracht. Die Reaktionstemperatur liegt zwischen − 30 und + 100 °C, vorzugsweise − 10 und + 30 °C. Als Mineralsäuren eignen sich vorzugsweise Halogenwasserstoffsäuren, wie Chlorwasserstoffsäure oder Bromwasserstoffsäure, sowie Schwefelsäure. Als Alkohole kommen vorzugsweise niedere aliphatische Alkohole, wie Methanol, Ethanol, n- und i-Propanol sowie Butanole in Frage. Mineralsäure und Alkohol werden mindestens in stöchiometrischer Menge in Bezug auf die Ausgangsverbindung der Formel IV, meist jedoch in der 1- bis 20-fachen stöchiometrischen Menge, eingesetzt. Nach Reaktionszeiten von 4 bis 48 Stunden werden die Reaktionsansätze mit Wasser vermischt. Die aus der organischen Phase, gegebenenfalls nach Verdampfen des inerten Verdünnungsmittels isolierten N-Aminomethyl-halogenacetanilide der Formel I sind in vielen Fällen rein. Sie können, wenn nötig, z. B. durch Umkristallisation gereinigt werden.

Man erhält die substituierten N-Aminomethylhalogenacetatanilide der Formel IV durch Umsetzung von N-Halogenmethylhalogenacetaniliden der Formel

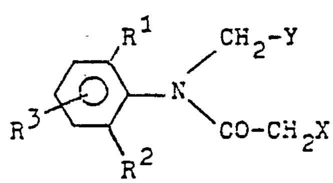

(II),

in der $R^1$, $R^2$, $R^3$ und X die oben angegebenen Bedeutungen haben und Y Halogen, z. B. Chlor, Brom, Jod, bedeutet, mit Cyanamiden der Formel

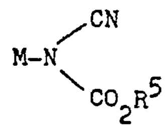

(III),

in der $R^5$ die oben angegebene Bedeutung hat und M Wasserstoff, ein Alkalimetall oder einen Tetraalkylammoniumrest bedeutet, gegebenenfalls in Gegenwart eines Säurebindemittels und gegebenenfalls in Gegenwart eines gegenüber den Reaktionspartnern inerten Lösungsmittels.

Als Lösungsmittel kommen z. B. aromatische Kohlenwasserstoffe, wie Toluol, Xylol, halogenierte aliphatische oder aromatische Kohlenwasserstoffe, wie Chlorbenzol, Chloroform, Methylenchlorid, Ether, wie Tetrahydrofuran, Nitrile, wie Acetonitril, N,N-Dialkylamide wie Dimethylformamid, Sulfone, wie Dimethylsufoxid oder Gemische dieser Lösungsmittel in Betracht. Die Reaktionstemperatur liegt zwischen 0 °C und 150 °C, vorzugsweise zwischen 20 und 100 °C.

Setzt man Cyanamide der Formel III, in der M die Bedeutung Wasserstoff hat, um, empfiehlt es sich, ein säurebindendes Mittel zu verwenden. Als solche kommen tertiäre Amine, z. B. Triethylamin, Pyridin, substituierte Pyridine, oder anorganische Basen, z. B. Oxide und Hydroxide, Carbonate und Hydrogencarbonate von Alkalimetallen, in Betracht.

Pro Mol N-Halogenmethylhalogenacetanilid der Formel II setzt man mindestens 1 Mol, beispielsweise 1 bis 1,2 Mol Cyanamid der Formel III ein. Zur Isolierung der Verbindungen der Formel IV wird beispielsweise — gegebenenfalls nach Abfiltrieren des entstandenen Halogenids — das Filtrat eingedampft und der Rückstand in einem organischen, mit Wasser nicht mischbaren Lösungsmittel gelöst. Dann wird die organische Phase mit Wasser gewaschen, getrocknet und danach unter vermindertem Druck eingeengt. Das so erhaltene Endprodukt ist in vielen Fällen rein ; wenn nötig, kann es durch Umkristallisation oder Chromatographie an Kieselgel gereinigt werden.

Die als Ausgangsverbindungen verwendeten N-Halogenmethylhalogenacetanilide sind teilweise bekannt (DE-AS-15 42 950). Sie können in an sich bekannter Weise durch Umsetzung von entsprechend substituierten Phenylazomethinen mit Halogenacetylhalogeniden hergestellt werden.

Cyanamide der Formel III sind ebenfalls bekannt (DE-OS-24 74 453, DE-OS-17 95 849).

Als Beispiele für die als Ausgangsprodukte geeigneten Cyanamide sowie deren Salze seien im einzelnen genannt :

Cyancarbaminsäuremethylester oder dessen Natrium-, Kalium- oder Tetramethylammoniumsalz,
Cyancarbaminsäureethylester oder dessen Natrium-, Kalium- oder Tetramethylammoniumsalz,
Cyancarbaminsäure-n-propylester oder dessen Natrium-, Kalium- oder Tetramethylammoniumsalz,
Cyancarbaminsäure-isopropylester oder dessen Natrium- oder Kaliumsalz,
Cyancarbaminsäure-n-butylester oder dessen Natrium- oder Kaliumsalz,
Cyancarbaminsäure-sec.-butylester oder dessen Natrium- oder Kaliumsalz,
Cyancarbaminsäure-n-hexylester oder dessen Natrium- oder Kaliumsalz.

Das folgende Beispiel erläutert die Herstellung der N-Aminomethylhalogenacetanilide der Formel I.

## Beispiel

Eine Mischung aus 52,0 g N-Chlormethyl-2-chloressigsäure-2'-methyl-6'-ethyl-anilid, 29,2 g des Natriumsalzes des Cyancarbaminsäuremethylester und 0,5 g Benzyltriethylammoniumchlorid in 170 ml Methylenchlorid wird bei 25 °C 14 Stunden gerührt. Die organische Phase ergibt nach Waschen mit 200 ml Wasser, Trocknen und Verdampfen des Lösungsmittels und Anteigen des Rückstandes mit wenig Ether 46,5 g N-Methoxycarbonyl-N-[N'-(2-chloracetyl)-2'-methyl-6'-ethyl-anilino-methyl]-cyanamid von Fp. : 71 bis 73 °C.

$C_{15}H_{18}N_3O_3Cl$ M 325,5

| | | | |
|---|---|---|---|
| ber. : | C 55,64 | H 5,60 | N 12,98 |
| gef. : | C 55,8 | H 6,00 | N 12,6 |

In eine Lösung von 32,4 g N-Methoxycarbonyl-N-[N'-(2-chloracetyl)-2'-methyl-6'-ethyl-anilino-methyl]-cyanamid in 19,2 g Methanol und 200 ml Methylenchlorid wird bei 0 bis 5 °C Chlorwasserstoffgas bis zur Sättigung (ca. 2 Stunden) eingeleitet. Nach 20-stündigem Stehen des verschlossenen Reaktionsansatzes bei gleicher Temperatur wird nach Zusatz von 100 ml Wasser eine Stunde bei 25 °C gerührt. Aus der organischen Phase isoliert man nach Waschen mit Wasser, Natriumbicarbonat-Lösung und Wasser sowie Umkristallisieren aus Methanol 24,2 g 2-Chlor-2'-methyl-6'-ethyl-N-(N'-methoxycarbonyl-ureidomethyl)-acetanilid vom Fp. 139-141 °C (Verbindung Nr. 1).

$C_{15}H_{20}N_3O_4Cl$ M 341,5
ber.: C 52,71 H 5,90 N 12,29
gef.: C 53,2 H 6,0 N 12,4

Folgende Verbindungen der Formel I wurden beispielsweise in entsprechender Weise erhalten:

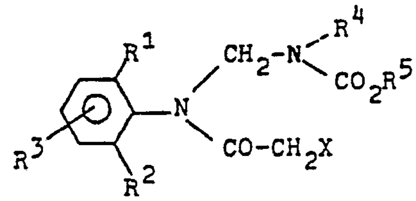

| Nr. | $R^1$ | $R^2$ | $R^3$ | $R^4$ | $R^5$ | X | Fp. [°C] |
|---|---|---|---|---|---|---|---|
| 2 | $CH_3$ | $CH_3$ | H | $CO-NH_2$ | $CH_3$ | Cl | 178-180 |
| 3 | $CH_3$ | $CH_3$ | H | $CO-NH_2$ | $C_2H_5$ | Cl | 181-183 |
| 4 | $C_2H_5$ | $C_2H_5$ | H | $CO-NH_2$ | $CH_3$ | Cl | 151-153 |

Folgende Verbindungen der Formel I können beispielsweise analog erhalten werden:

| Nr. | $R^1$ | $R^2$ | $R^3$ | $R^4$ | $R^5$ | X | Fp. [°C] |
|---|---|---|---|---|---|---|---|
| 5 | $CH_3$ | $CH_3$ | $3-CH_3$ | $CO-NH_2$ | $CH_3$ | Cl | |
| 6 | $CH_3$ | $CH_3$ | H | $CO-NH_2$ | $CH_3$ | Br | |
| 7 | $CH_3$ | $CH_3$ | H | $CO-NH_2$ | $n-C_3H_7$ | Cl | |
| 8 | $CH_3$ | $C_2H_5$ | H | $CO-NH_2$ | $C_2H_5$ | Cl | |
| 9 | $CH_3$ | $C_2H_5$ | H | $CO-NH_2$ | $CH_3$ | Br | |
| 10 | $CH_3$ | $C_2H_5$ | H | $CO-NH_2$ | $n-C_3H_7$ | Cl | |
| 11 | $CH_3$ | $C_2H_5$ | H | $CO-NH_2$ | $i-C_3H_7$ | Cl | |
| 12 | $C_2H_5$ | $C_2H_5$ | H | $CO-NH_2$ | $C_2H_5$ | Cl | |

Die Anwendung der Wirkstoffe der Formel I erfolgt z. B. in Form von direkt versprühbaren Lösungen, Pulvern, Suspensionen oder Dispersionen, Emulsionen, Öldispersionen, Pasten, Stäubemittel, Streumitteln, Granulaten durch Versprühen, Vernebeln, Verstäuben, Verstreuen, Beizen oder Gießen. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw. sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphtaline oder deren Derivate, z. B. Methanol, Ethanol, Propanol, Butanol, Chloroform, Tetrachlorkohlenstoff, Cyclohexanol, Cyclohexanon, Chlorbenzol, Isophoron usw., stark polare Lösungsmittel, z. B. Dimethylformamid, Dimethylsulfoxid, N-Methylpyrrolidon, Wasser usw. in Betracht. Die Salze können in Form ihrer wäßrigen Lösungen verwendet werden.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel

gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäuren, Phenolsulfonsäuren, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylen-octylphenolether, ethoxyliertes Isooctylphenol-, Octylphenol-, Nonylphenol, Alkylphenolpolyglykolether. Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver, Streu- und Stäubemittel können durch Mischen oder gemeinsames Vermahlen der wirksamen Substanzen mit einem festen Trägerstoff hergestellt werden. Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden wie Kieselsäuren, Silikate, Talkum, Kaolin, Kalk, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Die Zubereitungen enthalten 0,1 bis 90 Gew.%, vorzugsweise 1 bis 80 Gew.% Wirkstoff der Formel I. Beispiele für Formulierungen sind :

I. 20 Teile des Wirkstoffs Nr. 4 werden mit 12 Teilen Calciumsalz der Dodecylbenzolsulfonsäure, 8 Teile Fettalkoholpolyglykolether, 2 Teilen Natriumsalz eines Phenolsulfonsäure-harnstoff-kondensats, 68 Teilen eines paraffinischen Mineralöls innig vermischt. Man erhält eine stabile ölige Dispersion.

II. 3 Gewichtsteile des Wirkstoffs Nr. 1 werden mit 97 Gewichtsteilen feinteiligem Kaolin innig vermischt. Man erhält auf diese Weise ein Stäubemittel, das 3 Gewichtsprozent des Wirkstoffs enthält.

III. 40 Gewichtsteile des Wirkstoffs Nr. 2 werden mit 10 Teilen Natriumsalz eines Phenolsulfonsäureformaldehyd-kondensats, 2 Teilen Kieselsäuregel und 48 Teilen Wasser innig vermischt. Man erhält eine stabile wäßrige Dispersion. Durch Verdünnen mit 100 000 Gewichtsteilen Wasser erhält man eine wäßrige Dispersion.

IV. 20 Gewichtsteile des Wirkstoffs Nr. 4 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion.

Die Anwendung der Wirkstoffe bzw. ihrer Zubereitungen kann im Vorauflaufverfahren oder im Nachauflaufverfahren erfolgen. Vorzugsweise werden die neuen Wirkstoffe vor dem Auflaufen der unerwünschten Pflanzen, sowohl auf Kulturflächen als auch auf unbebautem Land, ausgebracht. Sind die Wirkstoffe für gewisse Kulturpflanzen weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder auf die unbedeckte Bodenfläche gelangen (post-directed, lay-by).

Die Aufwandmengen an Wirkstoff betragen je nach Jahreszeit und Wachstumsstadium 0,1 bis 15 kg/ha und mehr, vorzugsweise 0,25 bis 3 kg/ha.

Der Einfluß von Vertretern der Verbindungen der Formel I auf das Wachstum von erwünschten und unerwünschten Pflanzen wird anhand von Gewächshaus- und Freilandversuchen gezeigt :

## Gewächshausversuche

Als Kulturgefäße dienten Plastikblumentöpfe mit 300 $cm^3$ Inhalt und lehmigem Sand mit etwa 1,5 % Humus als Substrat. Die Samen der Testpflanzen wurden nach Arten getrennt flach eingesät. Unmittelbar danach erfolgte bei Vorauflaufbehandlung das Aufbringen der Wirkstoffe auf die Erdoberfläche. Sie wurden hierbei in Wasser als Verteilungsmittel suspendiert oder emulgiert und mittels fein verteilender Düsen gespritzt. Die Aufwandmenge entsprach 1,0 kg Wirkstoff/ha. Nach dem Aufbringen der Mittel wurden die Gefäße leicht beregnet, um Keimung und Wachstum in Gang zu bringen. Danach deckte man die Gefäße mit durchsichtigen Plastikhauben ab, bis die Pflanzen angewachsen waren. Diese Abdeckung bewirkte ein gleichmäßiges Keimen der Testpflanzen, sofern dies nicht durch die Wirkstoffe beeinträchtigt wurde.

Als Vergleichsmittel wurde das bekannte Herbizid

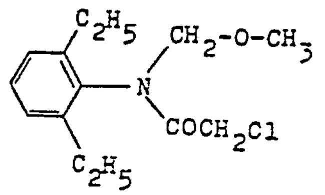

(A ; US-A-3 442 945)

ebenfalls mit 1,0 kg Wirkstoff/ha verwendet.

Die Versuchsgefäße wurden im Gewächshaus aufgestellt, wobei für wärmeliebende Arten wärmere Bereiche (20 bis 35 °C) und für solche gemäßigter Klimata 10 bis 25 °C bevorzugt wurden. Die Versuchsperiode erstreckte sich über 2 bis 4 Wochen. Während dieser Zeit wurden die Pflanzen gepflegt und ihre Reaktion auf die einzelnen Behandlungen ausgewertet. Bewertet wurde nach einer Skala von 0 bis 100. Dabei bedeutet 0 keine Schädigung oder normaler Ablauf und 100 kein Aufgang der Pflanzen bzw. völlige Zerstörung zumindest der oberirdischen Teile.

Freilandversuche

Es handelt sich um Kleinparzellenversuche auf Standorten mit lehmigem Sand von pH 6 und 1 bis 1,5 % Humusgehalt. Es wurden Vorauflaufanwendungen durchgeführt, welche unmittelbar bis spätestens 3 Tage nach der Saat erfolgten. Die Kulturpflanzen wurden in Reihen gesät. Die Unkrautflora war natürlich vorkommend. Die Substanzen wurden in Wasser als Träger- und Verteilungsmedium emulgiert oder suspendiert und mit Hilfe einer motorgetriebenen, auf einen Traktor montierten Parzellenspritze ausgebracht. Die Aufwandmengen der Verbindungen der Formel I variierten je nach Verbindung zwischen 0,25 und 2,0 kg Wirkstoff/ha.

Als Vergleichsmittel dienten dabei das bei den Gewächshausversuchen getestete Produkt A in den Aufwandmengen 0,25, 0,5 und 1,0 kg Wirkstoff/ha sowie das Herbizid der Formel

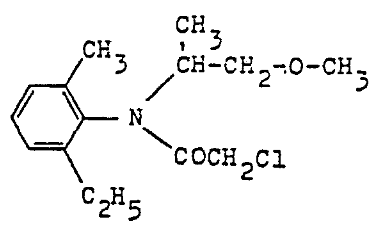

(B ; DE-A-23 28 340)

mit 2,0 kg Wirkstoff/ha.

Bei Fehlen natürlicher Niederschläge wurde beregnet, um Keimung und Wachstum von Nutzpflanzen und Unkräutern zu gewährleisten. Alle Versuche liefen über mehrere Wochen. In diesem Zeitraum wurde die Bewertung ebenfalls nach der Skala von 0 bis 100 vorgenommen.

In den Versuchen wurden die folgenden Pflanzenarten getestet :

Alopecurus myosuroides (Ackerfuchsschwanz), Avena fatua (Flughafer), Beta vulgaris (Zuckerrübe), Brassica napus (Raps), Bromus tectorum (Dach-Trespe), Chenopodium album (Weißer Gänsefuß), Echinochloa crus-galli (Hühnerhirse), Galinsoga spp. (Knopfkrautarten), Glycine max. (Soja), Gossypium hirsutum (Baumwolle), Sorghum halepense (Aleppohirse), Stellaria media (Vogelmiere), Zea mays (Mais), Matricaria spp. (Kamillearten), Lamium spp. (Taubnesselarten).

Bei der Prüfung auf herbizide Wirkung bei Vorauflaufanwendung von 1,0 kg/ha im Gewächshaus zeigte beispielsweise die Verbindung Nr. 4 eine besonders gute und im Vergleich zu der bekannten Verbindung A eine weit bessere Verträglichkeit für gewisse breitblättrige Kulturpflanzen. Hinsichtlich ihrer herbiziden Wirkung gegen unerwünschte Gräser verhielten sich beide Wirkstoffe ähnlich.

In Freilandversuchen zeichnete sich beispielsweise die Verbindung Nr. 1 im Vergleich zum Herbizid A bei Vorauflaufanwendung von 0,25 und 1,0 kg Wirkstoff/ha durch eine bessere Verträglichkeit und eine gesteigerte herbizide Aktivität gegen das Gras Echinochloa crus-galli aus.

Im Freiland zeigte sich, daß die Verbindung Nr. 1 bei Vorauflaufanwendung von 2,0 kg/ha eine bessere herbizide Wirkung gegen verschiedene breitblättrige unerwünschte Pflanzen hat als das Vergleichsmittel B und gleichzeitig ein hohes Maß an Verträglichkeit für Sojabohnen besitzt.

Die Verbindung Nr. 2 zeigte in den Freilandversuchen bei Vorauflaufanwendung von beispielsweise 0,5 kg Wirkstoff/ha bei guter Verträglichkeit für die Kulturpflanze Mais eine der Vergleichssubstanz A überlegene herbizide Wirkung.

Zur Verbreiterung des Wirkungsspektrums und zur Erzielung synergistischer Effekte können die

Verbindungen der Formel I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate und andere in Betracht.

Außerdem ist es nützlich, die Verbindungen der Formel I allein oder im Kombination mit anderen Herbiziden oder mit Antidots auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

**Ansprüche**

1. Substituierte N-Aminomethyl-halogenacetanilide der Formel

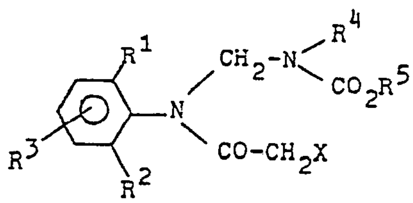

(I),

in der
$R^1$ $C_1$-$C_2$-Alkyl,
$R^2$ $C_1$-$C_2$-Alkyl,
$R^3$ Wasserstoff oder $C_1$-$C_4$-Alkyl,
$R^4$ den Rest —CO—$NH_2$,
$R^5$ $C_1$-$C_6$-Alkyl und
X Halogen bedeuten.

2. N-Aminomethyl-halogenacetanilid der Formel I gemäß Anspruch 1, dadurch gekennzeichnet, daß X Chlor bedeutet.

3. Verfahren zur Herstellung substituierter N-Aminomethyl-halogenacetanilide der Formel I gemäß Anspruch 1, dadurch gekennzeichnet, daß man N-Aminomethyl-halogenacetanilide der Formel

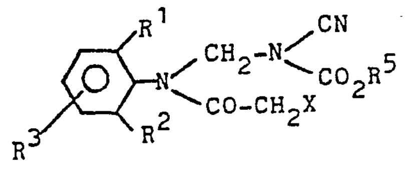

(IV),

in der $R^1$, $R^2$, $R^3$, $R^5$ und X die im Anspruch 1 genannten Bedeutungen haben, mit Mineralsäure in Gegenwart von Alkoholen verseift.

4. Herbizides Mittel, enthaltend ein N-Aminomethyl-halogenacetanilid der Formel

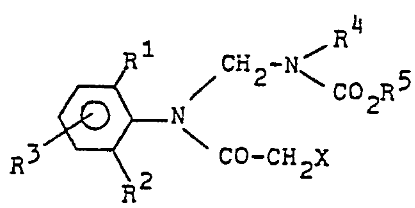

(I),

in der
$R^1$ $C_1$-$C_2$-Alkyl,
$R^2$ $C_1$-$C_2$-Alkyl,
$R^3$ Wasserstoff oder $C_1$-$C_4$-Alkyl,
$R^4$ den Rest —CO—$NH_2$,
$R^5$ $C_1$-$C_6$-Alkyl und
X Halogen bedeuten.

5. Herbizides Mittel, enthaltend inerte Zusatzstoffe und ein N-Aminomethyl-halogenacetanilid der Formel

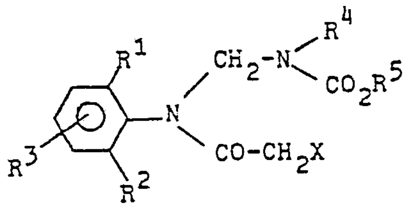

(I),

in der
$R^1$ $C_1$-$C_2$-Alkyl,
$R^2$ $C_1$-$C_2$-Alkyl,
$R^3$ Wasserstoff oder $C_1$-$C_4$-Alkyl,
$R^4$ den Rest —CO—$NH_2$,
$R^5$ $C_1$-$C_6$-Alkyl und
X Halogen bedeuten.

6. Herbizides Mittel, enthaltend inerte Zusatzstoffe und ein N-Aminomethyl-halogenacetanilid der Formel I gemäß Anspruch 1, wobei X Chlor bedeutet.

7. Verfahren zur Bekämpfung unerwünschten Pflanzenwuchses, dadurch gekennzeichnet, daß man die Pflanzen und/oder ihren Standort mit einer herbizid wirksamen Menge eines N-Aminomethyl-halogenacetanilids der Formel I gemäß Anspruch 1 behandelt.

**Claims**

1. A substituted N-aminomethylhaloacetanilide of the formula

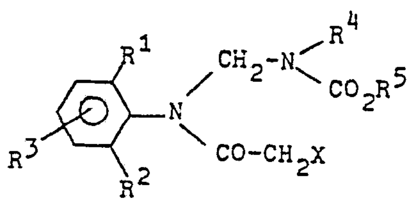

(I),

where
$R^1$ and $R^2$ are each $C_1$-$C_2$-alkyl,
$R^3$ is hydrogen or $C_1$-$C_4$-alkyl,
$R^4$ is —CO—$NH_2$,
$R^5$ is $C_1$-$C_6$-alkyl and
X is halogen.

2. An N-aminomethylhaloacetanilide of the formula I as claimed in claim 1, where X is chlorine.

3. A process for the preparation of a substituted N-aminomethylhaloacetanilide of the formula I as claimed in claim 1, wherein an N-aminomethylhaloacetanilide of the formula

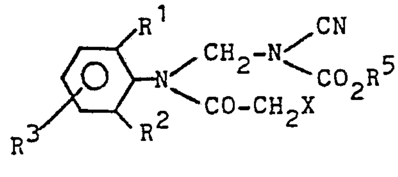

(IV),

where $R^1$, $R^2$, $R^3$, $R^5$ and X have the meanings given in claim 1, is hydrolyzed with a mineral acid in the presence of an alcohol.

4. A herbicidal agent containing an N-aminomethylhaloacetanilide of the formula

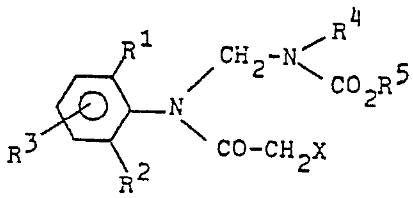

(I)

where
$R^1$ and $R^2$ are each $C_1$-$C_2$-alkyl,
$R^3$ is hydrogen or $C_1$-$C_4$-alkyl,
$R^4$ is —CO—$NH_2$,
$R^5$ is $C_1$-$C_6$-alkyl and
X is halogen.

5. A herbicidal agent containing inert additives and an N-aminomethylhaloacetanilide of the formula

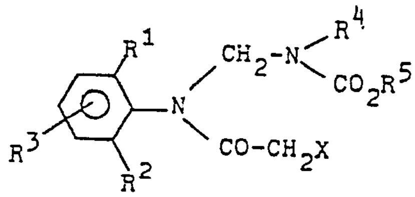

(I),

where
$R^1$ and $R^2$ are each $C_1$-$C_2$-alkyl,
$R^3$ is hydrogen or $C_1$-$C_4$-alkyl,
$R^4$ is —CO—$NH_2$,
$R^5$ is $C_1$-$C_6$-alkyl and
X is halogen.

6. A herbicidal agent containing inert additives and an N-aminomethylhaloacetanilide of the formula I as claimed in claim 1, where X is chlorine.

7. A process for combating unwanted plant growth, wherein the plants and/or their location are treated with a herbicidally effective amount of an N-aminomethylhaloacetanilide of the formula I as claimed in claim 1.

**Revendications**

1. N-Aminométhyl-halo-acétanilides substitués de la formule

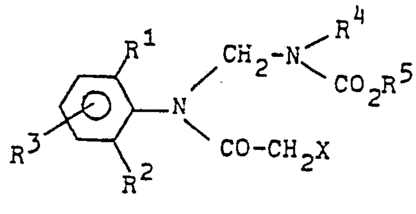

(I),

dans laquelle
$R^1$ = alkyle en $C_1$ ou $C_2$
$R^2$ = alkyle en $C_1$ ou $C_2$
$R^3$ = H ou alkyle en $C_1$ à $C_4$
$R^4$ = —CO—$NH_2$
$R^5$ = alkyle en $C_1$ et $C_6$ et
X = halogène.

2. N-Aminométhyl-halo-acétanilide de la formule I suivant la revendication 1, caractérisé en ce que X = Cl.

3. Procédé de préparation de N-aminométhyl-halo-acétanilides de la formule I suivant la revendication 1, caractérisé en ce que l'on soumet des N-aminométhyl-halo-acétanilides de la formule

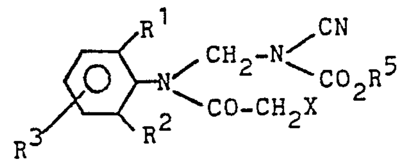

(IV),

dans laquelle $R^1$, $R^2$, $R^3$, $R^5$ et X possèdent les significations définies dans la revendication 1, à une saponification par un acide minéral en présence d'alcools.

4. Composition herbicide, contenant un N-aminométhyl-halo-acétanilide de la formule

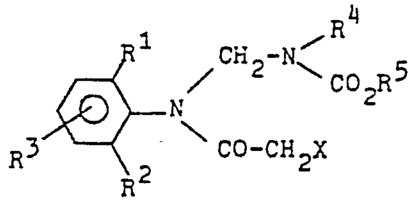

(I),

dans laquelle

$R^1$ = alkyle en $C_1$ ou $C_2$
$R^2$ = alkyle en $C_1$ ou $C_2$
$R^3$ = H ou alkyle en $C_1$ à $C_4$
$R^4$ = $-CO-NH_2$
$R^5$ = alkyle en $C_1$ à $C_6$ et
X = halogène.

5. Composition herbicide, comprenant des additifs inertes et un N-amino-méthyl-halo-acétanilide de la formule

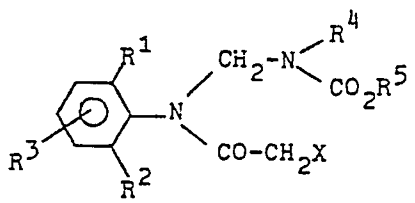

(I),

dans laquelle

$R^1$ = alkyle en $C_1$ ou $C_2$
$R^2$ = alkyle en $C_1$ ou $C_2$
$R^3$ = H ou alkyle en $C_1$ à $C_4$
$R^4$ = $-CO-NH_2$
$R^5$ = alkyle en $C_1$ à $C_6$ et
X = halogène.

6. Composition herbicide, comprenant des additifs inertes et un N-aminométhyl-halo-acétanilide de la formule I suivant la revendication 1, dans laquelle X = Cl.

7. Procédé de lutte contre la croissance de plantes indésirables, caractérisé en ce que l'on traite les plantes et(ou) leur biotope avec une quantité efficace d'un N-aminométhyl-halo-acétanilide de la formule I suivant la revendication 1.